# FASCICULE DE BREVET EUROPEEN

(11) **EP 3 997 055 B1**
(45) Date de publication et mention de la délivrance du brevet: **23.08.2023**
(21) Numéro de dépôt: 20736376.3
(22) Date de dépôt: 09.07.2020
(51) Int. Cl.: C07C 17/42, C07C 21/14, C07C 29/44, C07C 67/293, C07C 33/025, C07C 69/145

(54) **NOUVELLES FORMULATIONS STABLES DE COMPOSÉS 1-Z-BROMO ALCÈNES-1 ET LEUR UTILISATION DANS LA FABRICATION DE PHÉROMONES**
NEUE STABILE FORMULIERUNGEN VON 1-Z-BROMOALK-1-EN-VERBINDUNGEN UND IHRE VERWENDUNG ZUR HERSTELLUNG VON PHEROMONEN
NEW STABLE FORMULATIONS OF 1-Z-BROMOALK-1-ENE COMPOUNDS AND USE THEREOF IN THE MANUFACTURE OF PHEROMONES

(30) Priorité: 12.07.2019 FR 1907866
(43) Date de publication de la demande: 18.05.2022
(73) Titulaire: Melchior Material And Life Science France, 64170 LACQ (FR)
(72) Inventeur: GUERRET, Olivier, 46170 Pern (FR); GAYON, Eric, 64360 Monein (FR); GUILLONNEAU, Loic, 64000 Pau (FR)
(74) Mandataire: Regimbeau
(86) Numéro de dépôt international: PCT/EP2020/069399
(87) Numéro de publication internationale: WO 2021/009002

(56) Documents cités:
- GÉRARD CAHIEZ ET AL: "Iron Thiolate Complexes: Efficient Catalysts for Coupling Alkenyl Halides with Alkyl Grignard Reagents", CHEMISTRY - A EUROPEAN JOURNAL, vol. 18, no. 19, 29 mars 2012 (2012-03-29) , pages 5860-5863, XP055679211, DE ISSN: 0947-6539, DOI: 10.1002/chem.201200184
- GÉRARD CAHIEZ ET AL: "Gram-Scale, Cheap, and Eco-Friendly Iron-Catalyzed Cross-Coupling between Alkyl Grignard Reagents and Alkenyl or Aryl Halides", ORGANIC LETTERS, vol. 21, no. 8, 9 avril 2019 (2019-04-09), pages 2679-2683, XP055679215, ISSN: 1523-7060, DOI: 10.1021/acs.orglett.9b00665
- FUSINI GRAZIANO ET AL: "Identification and synthesis of new sex-specific components of olive fruit fly (Bactrocera oleae) female rectal gland, through original Negishi reactions on supported catalysts", TETRAHEDRON, ELSEVIER SCIENCE PUBLISHERS, AMSTERDAM, NL, vol. 74, no. 33, 6 juillet 2018 (2018-07-06), pages 4381-4389, XP085430938, ISSN: 0040-4020, DOI: 10.1016/J.TET.2018.07.003
- LARS ALLMENDINGER ET AL: "Total Synthesis of Sperabillin A and C", SYNLETT, no. 17, 1 janvier 2005 (2005-01-01), pages 2615-2618, XP055679244, DE ISSN: 0936-5214, DOI: 10.1055/s-2005-917106
- MURAHASHI S-I ET AL: "Stereoselective synthesis of alkenes and alkenyl sulfides from alkenyl halides using palladium and ruthenium catalysts", JOURNAL OF ORGANIC CHEMISTRY, AMERICAN CHEMICAL SOCIETY, US, vol. 44, no. 14, 1 janvier 1979 (1979-01-01), pages 2408-2417, XP002257406, ISSN: 0022-3263, DOI: 10.1021/JO01328A016

## Description

### DOMAINE DE L'INVENTION

La présente invention concerne de nouvelles compositions stables de composés 1-Z-bromo alcènes-1 de formule générale A :

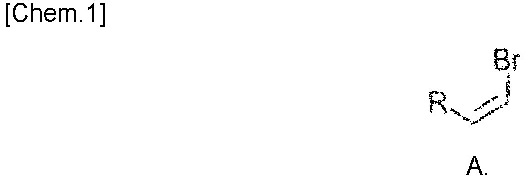

Ces compositions sont caractérisées en ce qu'elles comprennent en plus soit au moins un composé éther cyclique comprenant entre 4 et 6 atomes de carbone, soit au moins un éther de formule générale B : 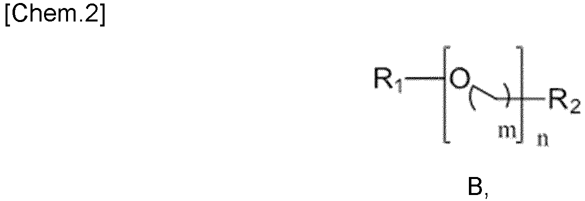 où R₁ et R₂ sont des radicaux carbonés ne comportant pas de fonction réactive vis-à-vis des organomagnésiens ni des organolithiens.

### CONTEXTE DE L'INVENTION

Les composés Z-bromovinyliques sont des intermédiaires clés pour la fabrication de certaines molécules organiques (voir par exemple Allmendinger L. et al. Synlett 2005, 17, 2615 qui décrit la synthèse de sperabilline A et C à partir de (Z)-1-bromoprop-1-ène ; Fusini G. et al. Tetrahedron 2018, 74, 4381 qui décrit la synthèse de sémiochimiques issus de *Bactrocera oleae*) et en particulier pour la fabrication de phéromones sexuelles de lépidoptères. Les phéromones sexuelles de lépidoptères sont des molécules organiques linéaires insaturées de 8 à 20 atomes de carbone, porteuses de doubles liaisons dont le nombre va de 1 à 3, conjuguées ou non. Ces molécules sont en outre terminées par une fonction polaire de type alcool, aldéhyde ou acétate.

La particularité de chaque phéromone sexuelle réside dans le fait que le signal phéromonal émis par un insecte doit être spécifique de ce seul insecte. Cette spécificité est rendue possible par l'ensemble des caractéristiques chimiques suivantes pour une molécule de phéromone donnée :
- longueur de chaine linéaire ; normalement ces chaines comportent un nombre pair d'atomes de carbone ;
- fonction terminale choisie parmi les fonctions alcool, aldéhydes et acétate ;
- nombre et position des doubles liaisons, certaines phéromones de lépidoptères se différenciant entre elles uniquement par le remplacement d'une double liaison par une triple liaison ; et
- stéréochimie de chaque double liaison.

Par ailleurs, pour un signal phéromonal donné, plusieurs de ces molécules peuvent être associées en un bouquet phéromonal dans des proportions variables, ce qui multiplie les possibilités de spécificité de signal phéromonal.

Par exemple, si on considère la famille des dérivés du 11-hexadecénol, nous constatons que :
- Le Z-11-hexadecénol et son acétate constituent les 2 composants du bouquet phéromonal de la sésamie du maïs qui fait des ravages tant en Europe qu'en Afrique, alors que son isomère E n'y contribue pas significativement.
- Le Z-11-hexadécénal est d'une part le composant majoritaire (80%) des phéromones de la pyrale du buis (en association avec son isomère E) ; il est aussi le composant principal (95%) des phéromones de la noctuelle de la tomate mais aussi du foreur du riz (chilo supressalis) alors que le E-hexadécénal ne contribue pas à leurs bouquets phéromonaux comme cela est référencé sur le site internet www.pherobase.com.

Un autre exemple est le bouquet phéromonal de la processionnaire du chêne qui est constitué principalement du (Z,Z)-11,13-hexadecadienyl acétate et dont l'isomère (E,Z) est un répulsif de l'insecte.

Ceci montre combien la sélectivité des structures chimiques que l'homme de l'art doit préparer est importante quand il s'agit de préparer une(les) phéromone(s) particulière(s) à un insecte (voir par exemple Quero et al. J. Chem. Ecology Food. Chem. 1995, 21, 1957-1969). Or, l'homme du métier a besoin de reproduire de manière synthétique ces phéromones de manière à pouvoir les utiliser dans le cadre de luttes biologiques contre des ravageurs des cultures.

Dans le cadre de la préparation de phéromones sexuelles de lépidoptères, qui sont pour rappel des molécules organiques linéaires de 8 à 20 atomes de carbone, porteuses de doubles liaisons dont le nombre va de 1 à 3, conjuguées ou non, l'homme du métier dispose de diverses méthodes de création de doubles liaisons dans de longues chaînes aliphatiques décrites dans l'art antérieur.

Les méthodes de choix pour créer des doubles liaisons dans des longues chaines aliphatiques sont d'abord les réactions de Wittig ou de Wittig Horner. De nombreux exemples de l'utilisation de telles réactions existent dans la littérature (voir par exemple les brevets EP0630877, US 5395993, US 6838576, EP0241335B1). Ces réactions entre un aldéhyde et un ylure de phosphore conduisent à des sélectivités Z/E comprises entre 80/20 et 92/8.

Une autre méthode de choix consiste au couplage de vinyliques halogénés avec un réactif de Grignard ou un organolithien ou d'un alcynure en présence d'un catalyseur métallique (voir par exemple Cahiez, G.; Avedissian, H. Synthesis 1998, 1199 ; Cahiez, G. & al. Chem. Eur. J. 2012, 18, 5860 ; Cahiez, G. et al. Org. Lett. 2019, 21, 2679 ; Murahashi, S.-I. et al. J. Org. Chem. 1979, 14, 2408). Cette méthode est beaucoup plus stéréosélective puisqu'elle retient la configuration du composé halogéné de départ : si l'homme du métier possède un composé vinylique halogéné exclusivement dans la configuration Z, l'oléfine obtenue après couplage aura une configuration équivalente (Quero et al. J. Chem. Ecology Food. Chem. 1995, 21, 1957-196). Les meilleurs rendements sont obtenus quand le composé vinylique est un composé bromé.

Cette dernière méthode présente l'avantage de ne pas générer des effluents phosphores.

Cependant, la difficulté de la transposition industrielle de cette dernière méthode réside surtout dans l'accès à des produits composés vinyliques bromés dont le ratio isomérique Cis/Trans serait suffisamment stable pour qu'ils soient conservés dans des conditions économiques jusqu'à leur utilisation en tant qu'intermédiaires pour la synthèse de molécules organiques telles que ces phéromones, d'autant que leur utilisation peut se faire sur un site industriel différent de celui qui a vu leur production. Par ailleurs, la synthèse de certaines molécules organiques telles que les phéromones sexuelles de lépidoptères nécessite l'utilisation d'organomagnésien ou d'organolithien qui sont connus pour réagir par addition nucléophile avec les époxydes, les cétones et les cétones insaturées 1-4 et par réaction acido-basique avec les alcools.

Il existe donc un réel besoin de disposer de composés vinyliques bromés dont le ratio isomérique Cis/Trans soit suffisamment stable pour qu'il soit conservé jusqu'à l'utilisation desdits composés en tant qu'intermédiaires de synthèse et dont le milieu de conservation soit compatible avec une réaction ultérieure avec un organomagnésien ou un organolithien.

### ART ANTERIEUR

Des techniques de stabilisation de composés vinyliques halogénés ont été étudiées par le passé ; nous pouvons par exemple citer les brevets US2364588, US2355319 et US2376075. Cependant, ces trois brevets portent uniquement sur la stabilisation au sens large de butènes halogénés, sans spécification de la stéréochimie du composé à stabiliser ni de la stéréochimie du composé persistant ou obtenu après un temps de stockage.

Ces trois brevets ne permettent donc pas à l'homme du métier de trouver une solution de stabilisation de la stéréochimie de composés 1-Z-bromo alcène. De plus, ils ne traitent pas de la problématique de disposer d'un milieu de conservation qui soit compatible avec une réaction ultérieure avec un organomagnésien ou un organolithien.

### RESUME DE L'INVENTION

Dans le cadre de la présente invention, les inventeurs ont découvert de manière surprenante que, dans une composition comprenant des composés 1-Z-bromo alcène, la présence de 0.5% à 30% en poids d'un éther non réactif vis-à-vis des organomagnésiens ou des organolithiens, par rapport au poids total de la composition, permet de préserver la stéréochimie dudit composé 1-Z-bromo alcène dans le temps et de rendre ainsi exploitable industriellement des composés 1-Z-bromo alcène.

Un autre intérêt de ces compositions est qu'elles sont compatibles avec leur utilisation ultérieure de couplage avec un composé organomagnésien ; les composés organomagnésiens sont généralement eux aussi stabilisés par des éthers. Un autre objet de l'invention est donc l'utilisation de ces compositions pour la fabrication de phéromones porteuses de doubles liaisons via des couplages organométalliques en présence d'organomagnésiens ou d'organolithiens.

Selon un premier aspect, la présente invention porte donc sur une composition comprenant :
- un 1-Z-Bromo alcène-1 de formule générale A: 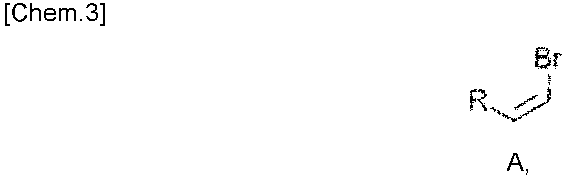 où R est un groupement alkyle linéaire comportant entre 1 et 24 atomes de carbones ; et
- soit au moins un éther cyclique dont le cycle comprend entre 4 et 6 atomes de carbone, éventuellement substitué par un groupement alkyle en C1-C3, de préférence par un méthyle ;
- soit au moins un éther de formule générale B : 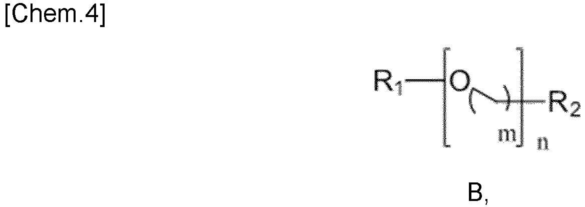 où n est compris entre 1 et 4 et m est compris entre 0 et 5, particulièrement entre 1 et 5, de préférence m est compris entre 1 et 3 ;
   et R1 et R2, identiques ou différents sont choisis dans le groupe comprenant : un groupement alkyle linéaire ou ramifié comportant 1 à 8 atomes de carbone,
   ladite composition étant caractérisée en ce que la teneur en 1-Z-Bromo alcène est comprise entre 70 et 99.5% en poids, de préférence entre 90 et 99% en poids et de manière encore préférentielle entre 93 et 98% en poids, par rapport au poids total de ladite composition.

Selon un deuxième aspect, la présente invention porte sur l'utilisation d'une composition selon l'invention dans une réaction de couplage du composé 1-Z-Bromo alcène-1 de formule générale A avec un composé choisi dans le groupe comprenant les organomagnésiens et les organolithiens.

Selon un troisième aspect, la présente invention porte sur l'utilisation d'une composition selon l'invention pour la fabrication de phéromone à chaine grasse.

### DESCRIPTION DETAILLEE DE L'INVENTION

Au sens de la présente invention, par « milieu de conservation qui soit compatible avec une réaction ultérieure avec un organomagnésien ou un organolithien », on entend un milieu de conservation dont seul(s) le(s) composé(s) 1-Z-Bromo alcène-1 de formule générale A est(sont) susceptible(s) de réagir avec des organomagnésiens ou des organolithiens.

Au sens de la présente invention, par « organomagnésien », on entend un composé organique présentant une liaison carbone-magnésium. La famille des organomagnésiens inclut de manière non limitative les réactifs de Grignard, qui sont des organomagnésiens mixtes de formule R'-MgX, où R' est une chaîne carbonée et X un halogène, et en particulier les composés de formule générale Y-(CH₂)ₙ-MgX où n est un nombre entier compris entre 2 et 20, de préférence n est compris entre 6 et 12 et où Y est une fonction éther, alcoolate (-OMgX), acétylate (-OC(O)CH₃) ou acétal ( 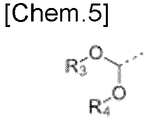 Ou 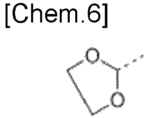 avec R3 et R4 étant des chaines alkyles en C1-C8, identiques ou différentes, de préférence R3 et R4 sont choisis parmi les groupements méthyl, éthyl ou propyl).

Au sens de la présente invention, par « organolithien », on entend un composé organométallique présentant une liaison carbone-lithium. Les organolithiens incluent de manière non limitative les alkyles lithium tels que le méthyllithium, le butyllithium ou l'hexyllithium ou encore les composés de formule générale R"-Li, où R" est une chaîne carbonée, et en particulier les composés de formule générale Y-(CH₂)ₙ-Li où n est un nombre entier compris entre 2 et 20, de préférence n est compris entre 6 et 12, et où Y est une fonction éther, alcoolate (-OLi), acétylate (-OC(O)CH₃) ou acétal ( 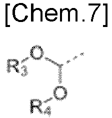 ou 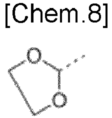 avec R3, R4 chaines alkyles en C1-C8, identiques ou différentes, de préférence choisis parmi les groupements méthyl, éthyl ou propyl).

Au sens de la présente invention, par « hydrogène labile », on entend un atome d'hydrogène ayant un caractère acide tel que par exemple, de manière non limitative, l'hydrogène des alcools, des acides carboxyliques, des amines, et des amides primaires et secondaires. Ainsi, un éther qui ne comprend pas d'hydrogène labile n'est pas susceptible de générer des protons.

Un premier objet de la présente invention est une composition comprenant :
- un 1-Z-Bromo alcène-1 de formule générale A: 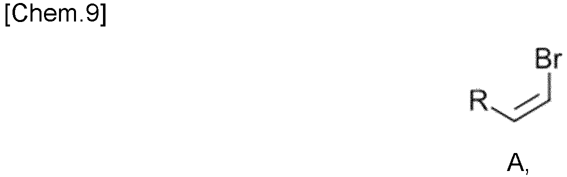 où R est un groupement alkyle linéaire comportant entre 1 et 24 atomes de carbones ; et
- soit au moins un éther cyclique dont le cycle comprend entre 4 et 6 atomes de carbone, éventuellement substitué par un groupement alkyle en C1-C3, de préférence par un méthyle ;
- soit au moins un éther de formule générale B : 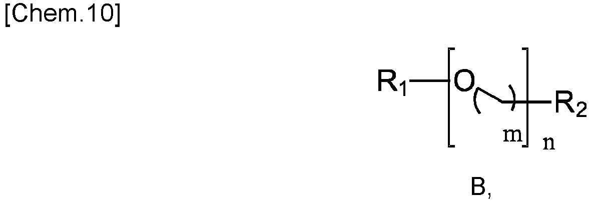
   où n est compris entre 1 et 4 et m est compris entre 0 et 5, en particulier entre 1 et 5, de préférence m est compris entre 1 et 3 ;
   et R1 et R2, identiques ou différents sont choisis dans le groupe comprenant : un groupement alkyle linéaire ou ramifié comportant 1 à 8 atomes de carbone,
   ladite composition étant caractérisée en ce que la teneur en 1-Z-Bromo alcène est comprise entre 70 et 99.5% en poids, de préférence entre 90 et 99% en poids et de manière encore préférentielle entre 93 et 98% en poids, par rapport au poids total de ladite composition.

Selon un mode de réalisation préféré, l'éther cyclique est choisi parmi le tétrahydrofurane, le méthyl tetrahydrofurane et le dioxane.

Selon un mode de réalisation, le groupement R du composé de formule générale A est choisi dans le groupe comprenant les groupements méthyl, éthyl, propyl, butyl, pentyl, hexyl, heptyl, octyl, nonyl, décanyl, undécanyl, dodécanyl, tridécanyl, tétradécanyl, pentadécanyl, hexadécanyl, heptadecanyl et octadecanyl.

Selon un mode de réalisation, les groupements R1 et R2 du composé de formule générale B, identiques ou différents sont choisis dans le groupe comprenant les groupements méthyl, éthyl, isopropyl, ter-butyl et le néopentyl. Avantageusement le composé B est le méthyl tert-butyl éther.

La teneur en 1-Z-Bromo alcène-1 dans la composition selon l'invention est comprise entre 70 et 99.5% en poids, de préférence entre 90 et 99% en poids et de manière encore préférentielle entre 93 et 98% en poids, par rapport au poids total de ladite composition.

Selon un mode de réalisation, la teneur en éther dans la composition selon l'invention est comprise entre 0.5 et 30% en poids, de préférence entre 1 et 10% en poids et de manière encore préférentielle entre 3 et 10% en poids, par rapport au poids total de ladite composition.

Un deuxième objet de la présente invention est l'utilisation de la composition selon l'invention dans une réaction de couplage d'un composé de formule générale A avec un composé choisi dans le groupe comprenant les organomagnésiens et les organolithiens.

Selon un mode de réalisation, l'invention porte sur l'utilisation de la composition selon l'invention dans une réaction de couplage d'un composé de formule générale A avec un composé organomagnésien de formule R'-MgX, où R' est une chaîne carbonée et X un halogène. Selon un mode de réalisation préféré, ledit composé organomagnésien a pour formule générale Y-(CH₂)ₙ-MgX où n est un nombre entier compris entre 2 et 20, de préférence n est compris entre 6 et 12, et où X est un atome de chlore ou de brome et où Y est une fonction éther, alcoolate (-OMgX), acétylate (-OC(O)CH₃) ou acétal ( 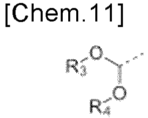 Ou 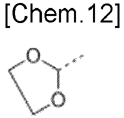 avec R3 et R4 sont des chaines alkyles C1-C8, identiques ou différentes, de préférence R3 et R4 sont choisis parmi les groupements méthyl, éthyl et propyl).

De préférence, la réaction a lieu en présence d'un catalyseur.

Selon un autre mode de réalisation, l'invention porte sur l'utilisation de la composition selon l'invention dans une réaction de couplage d'un composé de formule générale A avec un composé organolithien de formule générale Y-(CH₂)ₙ-Li où n est un nombre entier compris entre 2 et 20, de préférence n est compris entre 6 et 12 et où Y est une fonction éther, alcoolate (-OLi), acétylate (-OC(O)CH₃) ou acétal ( 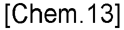 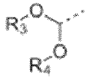 Ou 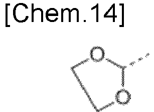 avec R3 et R4 sont des chaines alkyles en C1-C8, identiques ou différentes, de préférence R3 et R4 sont choisis parmi les groupements méthyl , éthyl et propyl).

De préférence, la réaction a lieu en présence d'un catalyseur.

Un troisième objet de la présente invention est l'utilisation d'une composition selon l'invention pour la fabrication de phéromone à chaine grasse.

Selon un mode de réalisation, l'invention porte sur l'utilisation de la composition selon l'invention pour la fabrication de phéromone de lépidoptère porteuse d'au moins une insaturation dans une configuration Z via une réaction de couplage en présence d'un organomagnésien ou d'un organolithien et d'un complexe métallique.

Selon un mode de réalisation, les compositions selon l'invention sont utilisées pour la synthèse de composés choisis parmi : Z-7-décén-1-yl acétate, Z-9-dodecen-1-yl acétate, Z-9-dodecen-1-ol, Z-11-tetradécén-1-ol, Z-11-tetradécén-1-ol, Z-11-tetradecen-1-yl acétate, Z-11-tetra-décénal, (Z)-hexadec-13-en-11-yn -1-yl acétate, (Z,Z)-11,13-hexadecadien-1-yl-acetate et (Z,Z)-1 1,13-hexadecadienal.

Selon un mode de réalisation, les compositions selon l'invention sont utilisées pour la synthèse de composés choisis parmi : Z-5-decen-1-ol, Z-5-decen-1-yl acétate, Z-7-dodecen-1-ol, Z-7-dodecen-1-yl acétate , Z-9-tetradecen-1-ol, Z-9-tetradecen-1-yl acétate, Z-9-tetradecenal , Z -11-hexadecen-1-ol, Z-11-hexadecen-1-yl acétate , Z-11-hexadecenal , Z-13-octadecen-1-yl acétate et Z-13-octadecenal.

Les exemples qui suivent visent à illustrer la présente invention de manière non limitative et permettront de mieux comprendre l'invention et sa portée.

### EXEMPLES

La synthèse des acides α,β di-bromés précurseurs des composés selon l'invention sont obtenus selon le mode opératoire décrit dans la publication K. Mori, J.-L. Brevet, Synthesis, 1991, 1125.

La méthode analytique consiste en une analyse par chromatographie en phase gaz (GC) sur un appareil HP 5890 Series II. La colonne chromatographique est une colonne Optima delta 6,30 m, 0,25 mm, 0,25 µm.

Le four suit le profil de température suivant : Température initiale : 40°C ; Temps initial 5 min ; Gradient 5°/min ; Température finale : 125°C ; Durée 15 min.

La température de l'injecteur est 250°C, celle du détecteur vaut 280°C, le volume injecté est de 1 µL et la pression est de 6psi. La concentration de l'échantillon est de 75 g/L dans le tétrahydrofurane (THF).

Les réactions sont réalisées dans un réacteur de 20 L en verre à double enveloppe et les distillations sont effectuées au moyen d'une colonne verre de 10 plateaux théoriques.

### Exemple 1 : Préparation du 1-Z-Bromo butène-1

Dans un réacteur propre préalablement purgé à l'azote, on introduit successivement :
- de l'acide 2,3-dibromopentanoïque : 3,830 kg présentant une pureté massique de 86,6% (1 eq) ; et
- du diméthylformamide (DMF) : 7,660 L (2 vol).

Le fluide réfrigérant est refroidi à -15°C. La solution est ensuite chauffée à 55°C, puis on ajoute une solution aqueuse à 50% massique de soude selon un débit permettant de contrôler le CO₂ produit par la réaction de décarboxylation et de maintenir la température du milieu réactionnel entre 53° et 80°C.

A la fin de l'addition de la solution aqueuse à 50% massique de soude, la température du réacteur est augmentée jusqu'à 110°C d'abord sous pression atmosphérique, puis sous pression réduite à 100 mbars de manière à entrainer l'eau et les dernières traces de produit.

Les bonnes fractions de distillation sont ensuite rassemblées et le solvant organique (DMF) est éliminé par des lavages aqueux.

On obtient 1,414 kg de 1-Z-Bromo butène-1 avec un excès isomérique 99.8/0.2 et une pureté massique >99%. La teneur en eau mesurée par titration de Karl Fischer est inférieure à 0.15%.

### Exemple 2 : Préparation du 1-Z-bromohexène-1

Dans un réacteur propre préalablement purgé à l'azote, on introduit successivement :
- de l'acide 2,3-dibromoheptanoïque : 3,5 kg présentant une pureté massique de 88% (1 eq) ; et
- du diméthylformamide (DMF) : 6,3 L (2 vol).

Le fluide réfrigérant est refroidi à -15°C.

La solution est ensuite chauffée à 55°C, puis on ajoute une solution aqueuse à 50% massique de soude selon un débit permettant de contrôler le CO₂ produit par la réaction de décarboxylation et de maintenir la température du milieu réactionnel entre 53° et 80°C.

A la fin de l'addition de la solution aqueuse à 50% massique de soude, la température du réacteur est augmentée jusqu'à 110°C d'abord sous pression atmosphérique, puis sous pression réduite à 100 mbars de manière à entrainer l'eau et les dernières traces de produit.

Les bonnes fractions de distillation sont ensuite rassemblées et le solvant organique (DMF) est éliminé par des lavages aqueux.

On obtient 1,175 kg de 1-Z-Bromo hexène-1 avec un excès isomérique 99.7/0.3 et une pureté massique >99%.

### Exemple 3 : Préparation de compositions selon l'invention

On ajoute au 1-Z-Bromo butène-1 de l'exemple 1 (noté C4) ou au 1-Z-bromohexène-1 de l'exemple 2 (noté C6), une proportion (en %) d'un éther et on étudie la stabilité du ratio Z/E en considérant le nombre de jours à partir duquel le ratio Z/E atteint un taux inférieur à 95% d'isomère Z dans des conditions de vieillissement à température contrôlée (noté T95). Les données sur les compositions étudiées sont rassemblées dans le tableau suivant :

**[Table 1]**

| N° de formulation | 1 | 2 | 3 | 4 | 5 | 6 | 7 | 8 | 9 | 10 |
|---|---|---|---|---|---|---|---|---|---|---|
| Bromoalcène | C4 | | | | | C6 | | | | |
| Ether ajouté | aucun | THF | MTBE | MTBE | MTBE | Aucun | THF | MTBE | MTBE | MTBE |
| % poids d'éther | 0 | 5 | 3 | 45 | 9 | 0 | 5 | 3 | 4 | 9 |
| Ratio initial Z/E | 97.1/2.9 | 99.8/0.2 | | | | 99.7/0.3 | | | | |
| Température de stockage (°C) | 4 | 25 | 25 | 25 | 4 | 4 | 25 | 25 | 25 | 4 |
| T95 (jours) | 3 | >85 | >85 | 160 | >203 | 20 | >85 | >85 | >85 | >85 |

### MTBE = méthyl tert-butyl éther

Les inventeurs ont ainsi mis en évidence que les compositions sans éther présentent un ratio isomérique qui passe de 97.1/2.9 à 95/5 à 4°C en 3 jours dans le cas du 1-Z-bromo butène-1 et de 99.7/0.3 à 95/5 à 4°C en 20 jours dans le cas du 1-Z-bromohexène-1. En revanche, toutes les compositions contenant une proportion même limitée d'un éther conduisent à des stabilités stéréochimiques sur des durées supérieures à 30 jours à 25°C, ce qui les rend utilisables dans un processus de synthèse multi-étapes.

Le THF et le MTBE choisis pour cet exemple sont des solvants classiques pour fabriquer ou utiliser des organomagnésiens ou des organolithiens.

### Exemple 4 : Préparation de composition hors invention et leur stabilité

Au 1-Z-Bromo butène-1 de l'exemple 1, on ajoute soit 5% d'un solvant de type alcène (amylène), soit du toluène, soit un solvant chloré (dichlorométhane).

Dans les cas des compositions comprenant le solvant de type alcène (amylène) ou le toluène, les T95 sont respectivement de 9 et 10 jours.

Dans le cas de la composition comprenant le solvant chloré (dichlorométhane), le T95 est supérieur à 20 jours mais le dichlorométhane introduit réagit violemment avec les organolithiens et les organomagnésiens.

### Exemple 5 : Utilisation de la composition 9 de l'exemple 3 en réaction avec du BrMaO-(CH₂)₁₀-MgBr pour la fabrication de Z-11-hexadécenol

Dans un tétracol sec de 250 mL équipé d'un thermomètre et d'une agitation mécanique, on introduit, sous argon à 25 °C, 44mL d'une solution à 1mol/L de l'organomagnésien BrMgO-(CH₂)₁₀-MgBr obtenu à partir de chlorodécanol. Ensuite, on ajoute 45 mL de THF. Le mélange est refroidi à - 5 °C.

L'acétylacétonate de fer(III) est ajouté en une portion (14 mg). Les parois du tétracol sont rincées avec 4 mL de THF. Le (Z)-bromohex-1-ène (6.52g) est ensuite introduit en 5 minutes en conservant une température comprise entre - 5 °C et 0 °C. L'agitation est maintenue à cette température pendant 20 minutes.

On ajoute 80 mL d'une solution de HCl 1 mol/L en conservant une température inférieure à 30 °C. Les deux phases obtenues sont séparées : la phase aqueuse est extraite avec 3x20 mL d'éther de pétrole ; les phases organiques sont combinées, séchées sur MgSO₄, filtrées et concentrées à l'évaporateur rotatif. L'huile résiduelle est distillée sous vide. Température d'ébullition à 0.04mmHg = 118 °C.

On obtient alors 8.6 g d'une huile incolore caractérisée composée d'un mélange des isomères Z et E du 11-hexadécénol (89% de rendement) présentant un ratio isomérique Z/E de 97/3.

### Exemple 6 : Utilisation de la composition 5 de l'exemple 3 pour la synthèse de la phéromone de la processionnaire du pin ((Z)-hexadec-13-en-11-yn -1-yl acetate)

Dans un tricol de 250 ml muni d'une agitation magnétique, à température ambiante, on charge 35mL de diéthylamine (Température : 19°C, milieu incolore) puis on inerte le milieu réactionnel par une séquence vide/azote.
- On prélève 10 mL de diéthylamine précédemment inertée pour solubiliser 10g de 11-dodecynol.
- On ajoute ensuite dans le tricol 20 mg de PdCl₂ (milieu orange) et 54 mg de CuCl (milieu bleu/violet). Après homogénéisation du milieu, on effectue un inertage vide/azote (le milieu devient vert, petite endothermie de 5°C).
- On introduit 9,7 g de solution de 1-bromobut-1-ène contenant 9% de THF, goutte à goutte dans le milieu.
- On ajoute alors goutte à goutte le docec-11-yn-1-ol en solution dans la diéthylamine.
- On maintient sous agitation à 60°C pendant 5h jusqu'à ce que le milieu prenne une teinte marron clair.

A la fin de la réaction, on ajoute 50 mL d'une solution 0.1N d'acide chlorhydrique puis on effectue plusieurs extractions au méthyl cyclohexane. Après évaporation de ce solvant on obtient 12.6g d'acétate de (Z)-hexadec-13-en-11-yn -1-yl à 97% avec un ratio isomérique Z/E de 98/2.

## Revendications

1. Composition comprenant :
- un 1-Z-Bromo alcène-1 de formule générale A : 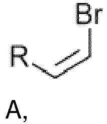 où R est un groupement alkyle linéaire comportant entre 1 et 24 atomes de carbones ; et
- soit au moins un éther cyclique dont le cycle comprend entre 4 et 6 atomes de carbone, éventuellement substitué par un groupement alkyle en C1-C3, de préférence par un méthyle ;
- soit au moins un éther de formule générale B : 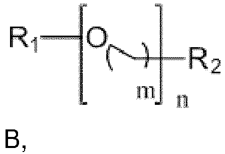 où n est compris entre 1 et 4 et m est compris entre 0 et 5, de préférence m est compris entre 1 et 3 ;
et R1 et R2, identiques ou différents sont choisis dans le groupe comprenant : un groupement alkyle linéaire ou ramifié comportant 1 à 8 atomes de carbone, ladite composition est **caractérisée en ce que** la teneur en 1-Z-Bromo alcène est comprise entre 70 et 99.5% en poids, de préférence entre 90 et 99% en poids et de manière encore préférentielle entre 93 et 98% en poids, par rapport au poids total de ladite composition.

2. Composition selon la revendication 1 **caractérisée en ce que** R est choisi dans le groupe comprenant les groupements méthyl, éthyl, propyl, butyl, pentyl, hexyl, heptyl, octyl, nonyl, décanyl, undécanyl, dodécanyl, tridécanyl, tétradécanyl, pentadécanyl, hexadécanyl, heptadecanyl et octadecanyl.

3. Composition selon les revendications 1 ou 2 **caractérisée en ce que** R1 et R2, identiques ou différents sont choisis dans le groupe comprenant les groupements méthyl, éthyl, isopropyl, ter-butyl et le néopentyl.

4. Composition selon l'une des revendications précédentes **caractérisée en ce que** l'éther cyclique est choisi dans le groupe comprenant le tétrahydrofurane, le méthyl tétrahydrofurane et le dioxane.

5. Composition selon l'une des revendications 1 à 4 telle que la teneur en éther est comprise entre 0.5 et 30% en poids de préférence entre 1 et 10% en poids et de manière encore préférentielle entre 3 et 10% en poids, par rapport au poids total de ladite composition.

6. Utilisation d'une composition selon l'une des revendications 1 à 5 dans une réaction de couplage d'un composé de formule générale A avec un composé choisi dans le groupe comprenant les organomagnésiens et les organolithiens.

7. Utilisation d'une composition selon la revendication 6 dans une réaction de couplage d'un composé de formule générale A avec un composé organomagnésien de formule générale Y-(CH2)n-MgX où n est un nombre entier compris entre 2 et 20, X est un atome de chlore ou de brome et où Y est une fonction éther, alcoolate choisie parmi - OMgX, acétylate-OC(O)CH3 ou acétal choisie parmi 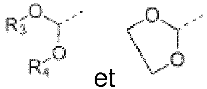 , avec R3 et R4 sont des chaines alkyles C1-C8, identiques ou différentes, de préférence R3 et R4 sont choisis parmi les groupements méthyl, éthyl et propyl.

8. Utilisation d'une composition selon la revendication 6 dans une réaction de couplage d'un composé de formule générale A avec un composé organolithien de formule générale Y-(CH2)n-Li où n est un nombre entier compris entre 2 et 20 et où Y est une fonction alcoolate OLi, acétylate -OC(O)CH3 ou acétal choisie parmi 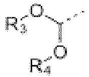 et 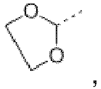 avec R3 et R4 sont des chaines alkyles en C1-C8, identiques ou différentes, de préférence R3 et R4 sont choisis parmi les groupements méthyl , éthyl et propyl.

9. Utilisation d'une composition selon l'une des revendications 1 à 5 pour la fabrication de phéromone à chaine grasse.

## Patentansprüche

1. Zusammensetzung, umfassend:
- Ein 1-Z-Bromalk-1-en der allgemeinen Formel A: 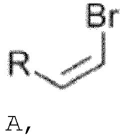 in der R eine lineare Alkylgruppe ist, die zwischen 1 und 24 Kohlenstoffatomen umfasst; und
- entweder mindestens einen cyclischen Ether, dessen Cyclus zwischen 4 und 6 Kohlenstoffatomen umfasst, gegebenenfalls substituiert durch eine C1-C3-Alkylgruppe, vorzugsweise durch Methyl;
- oder mindestens einen Ether der allgemeinen Formel B: 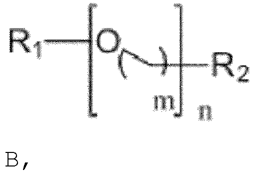
in der n zwischen 1 und 4 liegt und m zwischen 0 und 5 liegt, vorzugsweise m zwischen 1 und 3 liegt;
und R1 und R2, die gleich oder verschieden sind, ausgewählt sind aus der Gruppe umfassend: eine lineare oder verzweigte Alkylgruppe, die 1 bis 8 Kohlenstoffatome umfasst, wobei die Zusammensetzung **dadurch gekennzeichnet** ist, das der 1-Z-Bromalkengehalt zwischen 70 und 99,5 Gew.-%, vorzugsweise zwischen 90 und 99 Gew.-% und noch mehr bevorzugt zwischen 93 und 98 Gew.-% liegt, bezogen auf das Gesamtgewicht der Zusammensetzung.

2. Zusammensetzung nach Anspruch 1, **dadurch gekennzeichnet, dass** R aus der Gruppe umfassend Methyl-, Ethyl-, Propyl-, Butyl-, Pentyl-, Hexyl-, Heptyl-, Octyl-, Nonyl-, Decanyl-, Undecanyl-, Dodecanyl-, Tridecanyl-, Tetradecanyl-, Pentadecanyl-, Hexadecanyl-, Heptadecanyl- und Octadecanylgruppen ausgewählt ist.

3. Zusammensetzung nach den Ansprüchen 1 oder 2, **dadurch gekennzeichnet, dass** R1 und R2, die gleich oder verschieden sind, aus der Gruppe umfassend Methyl-, Ethyl-, Isopropyl-, tert.-Butyl- und Neopentylgruppen ausgewählt sind.

4. Zusammensetzung nach einem der vorhergehenden Ansprüche, **dadurch gekennzeichnet, dass** der cyclische Ether aus der Gruppe umfassend Tetrahydrofuran, Methyltetrahydrofuran und Dioxan ausgewählt ist.

5. Zusammensetzung nach einem der Ansprüche 1 bis 4, wobei der Ethergehalt zwischen 0,5 und 30 Gew.-%, vorzugsweise zwischen 1 und 10 Gew.-% und noch mehr bevorzugt zwischen 3 und 10 Gew.-% liegt, bezogen auf das Gesamtgewicht der Zusammensetzung.

6. Verwendung einer Zusammensetzung nach einem der Ansprüche 1 bis 5 in einer Reaktion zur Kupplung einer Verbindung der allgemeinen Formel A mit einer Verbindung, die aus der Gruppe umfassend Organomagnesium und Organolithium ausgewählt ist.

7. Verwendung einer Zusammensetzung nach Anspruch 6 in einer Reaktion zur Kupplung einer Verbindung der allgemeinen Formel A mit einer Organomagnesiumverbindung der allgemeinen Formel Y-(CH2)n-MgX, in der n eine ganze Zahl ist, die zwischen 2 und 20 liegt, X ein Chlor- oder Bromatom ist und in der Y eine Ether-, Alkoholat-, die aus -OMgX ausgewählt ist, Acetylat-OH(O)CH3- oder Acetalfunktion ist, die aus 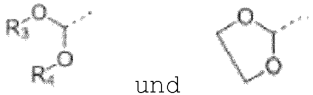 ausgewählt ist, wobei R3 und R4 C1-C8-Alkylketten sind, die gleich oder verschieden sind, vorzugsweise wobei R3 und R4 aus Methyl-, Ethyl- und Propylgruppen ausgewählt sind.

8. Verwendung einer Zusammensetzung nach Anspruch 6 in einer Reaktion zur Kupplung einer Verbindung der allgemeinen Formel A mit einer Organolithiumverbindung der allgemeinen Formel Y-(CH2)n-Li, in der n eine ganze Zahl ist, die zwischen 2 und 20 liegt und in der Y eine Alkoholatfunktion OLi, Acetalfunktion OH(O)CH3 oder Acetalfunktion ist, die aus 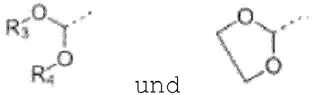 ausgewählt ist, wobei R3 und R4 C1-C8-Alkylketten sind, die gleich oder verschieden sind, vorzugsweise wobei R3 und R4 aus Methyl-, Ethyl- und Propylgruppen ausgewählt sind.

9. Verwendung einer Zusammensetzung nach einem der Ansprüche 1 bis 5 zur Herstellung von Pheromonen mit Fettketten.

## Claims

1. A composition comprising:
- a 1-Z-bromoalk-1-ene of general formula A: 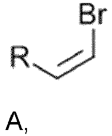 where R is a linear alkyl group having between 1 and 24 carbon atoms; and
- either at least one cyclic ether whose ring comprises between 4 and 6 carbon atoms, optionally substituted by a C1-C3 alkyl group, preferably by a methyl;
- or at least one ether of general formula B: 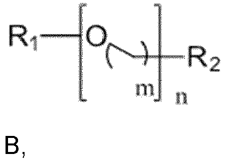
where n is comprised between 1 and 4 and m is comprised between 0 and 5, preferably m is comprised between 1 and 3;
and R1 and R2, identical or different, are selected from the group comprising: a linear or branched alkyl group having 1 to 8 carbon atoms, said composition is **characterized in that** the 1-Z-bromoalkene content is comprised between 70 and 99.5% by weight, preferably between 90 and 99% by weight and still more preferentially between 93 and 98% by weight, based on the total weight of said composition.

2. The composition according to claim 1, **characterized in that** R is selected from the group comprising methyl, ethyl, propyl, butyl, pentyl, hexyl, heptyl, octyl, nonyl, decanyl, undecanyl, dodecanyl, tridecanyl, tetradecanyl, pentadecanyl, hexadecanyl, heptadecanyl and octadecanyl groups.

3. The composition according to claims 1 or 2, **characterized in that** R1 and R2, identical or different, are selected from the group comprising methyl, ethyl, isopropyl, tert-butyl and neopentyl groups.

4. The composition according to one of the preceding claims, **characterized in that** the cyclic ether is selected from the group comprising tetrahydrofuran, methyl tetrahydrofuran and dioxane.

5. The composition according to one of claims 1 to 4, such that the ether content is comprised between 0.5 and 30% by weight, preferably between 1 and 10% by weight and still more preferentially between 3 and 10% by weight, based on the total weight of said composition.

6. Use of a composition according to one of claims 1 to 5 in a coupling reaction of a compound of general formula A with a compound selected from the group comprising organomagnesiums and organolithiums.

7. Use of a composition according to claim 6 in a coupling reaction of a compound of general formula A with an organomagnesium compound of general formula Y-(CH₂)ₙ-MgX where n is an integer comprised between 2 and 20, X is a chlorine or bromine atom and where Y is an ether function, alcoholate function selected from - OMgX, acetylate-OC(O)CH₃ or acetal function selected from 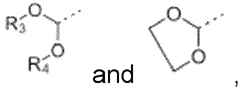 with R3 and R4 being C1-C8 alkyl chains, identical or different, preferably R3 and R4 are selected from methyl, ethyl and propyl groups.

8. Use of a composition as claimed in claim 6 in a coupling reaction of a compound of general formula A with an organolithium compound of general formula Y-(CH₂)ₙ-Li where n is an integer comprised between 2 and 20 and where Y is an alcoholate function -OLi, acetylate function -OC(O)CH₃ or acetal function selected from 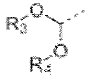 and 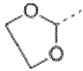 , with R3 and R4 being C1-C8 alkyl chains, identical or different, preferably R3 and R4 are selected from methyl, ethyl and propyl groups.

9. Use of a composition as claimed in one of claims 1 to 5 for the manufacture of fatty chain pheromone.
